# EUROPÄISCHE PATENTANMELDUNG

(11) **EP 1 958 627 A2**
(43) Veröffentlichungstag der Anmeldung: **20.08.2008**
(21) Anmeldenummer: 07123580.8
(22) Anmeldetag: 19.12.2007
(51) Int. Cl.: A61K 31/215, A61K 8/37, A61Q 11/00, A61Q 19/10

(54) **Verwendung bestimmter Menthyl-3-oxocarbonsäureester als physiologisch wirksame Kühlsubstanzen**

(30) Priorität: 04.01.2007 US 883400 P
(71) Anmelder: Symrise GmbH & Co. KG, 37603 Holzminden (DE)
(72) Erfinder: Schöning, Axel, 37603, Holzminden (DE); Wiedwald, Bernd, 37603, Holzminden (DE)
(74) Vertreter: Stilkenböhmer, Uwe Michael

(57) **Zusammenfassung**

Beschrieben wird die Verwendung einer Verbindung der Formel (I) oder (ent-I) oder einer Mischung bestehend aus zwei, drei oder mehr Verbindungen der Formel (I) oder (ent-I) 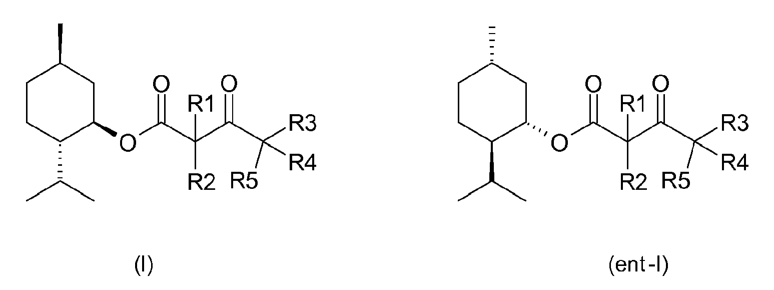 (a) als Kühlsubstanz für nicht-therapeutische Zwecke
oder
(b) zur Herstellung eines Arzneimittels,

wobei in jeder der Formeln (I) und (ent-I)
R1, R2, R3, R4 und R5 unabhängig voneinander jeweils Wasserstoff oder einen linearen, verzweigten oder cyclischen, gesättigten oder ungesättigten Kohlenwasserstoffrest mit 1 bis 4 Kohlenstoffatomen bedeuten.

## Beschreibung

Die vorliegende Erfindung betrifft die Verwendung bestimmter Menthyl-3-oxocarbonsäureester der weiter unten angegebenen Formeln (I) und/oder (ent-I) als (physiologisch wirksame) Kühlsubstanzen für nicht-therapeutische und therapeutische Zwecke sowie zur Herstellung von Arzneimitteln. Die Erfindung betrifft zudem die Verwendung entsprechender Mischungen sowie bestimmte neue Mischungen umfassend Verbindungen der weiter unten angegebenen Formeln (I) oder (ent-I). Zudem betrifft die Erfindung bestimmte Zubereitungen, welche eine zum Erreichen einer physiologischen Kühlwirkung auf der Haut und/oder Schleimhaut ausreichende Menge einer Verbindung der Formel (I) oder (ent-I) oder einer entsprechenden Mischung umfassen. Schließlich betrifft die Erfindung auch therapeutische und nicht-therapeutische Verfahren zum Erreichen einer physiologischen Kühlwirkung auf der Haut und/oder Schleimhaut.

Nachfolgend wird insbesondere der Begriff "Kühlsubstanz" zur Bezeichnung von physiologisch wirksamen Kühlsubstanzen (Kühlwirkstoffen) verwendet. Kühlsubstanzen werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als Kühlsubstanzen können sowohl Einzelkomponenten als auch Gemische verwendet werden.

Die bekannteste Kühlsubstanz ist L-Menthol, welches aber einige Nachteile besitzt, z. B. einen starken Geruchseindruck, eine hohe Flüchtigkeit und in höheren Konzentrationen einen bitteren und/oder scharfen Eigengeschmack. Der Einsatz von L-Menthol kann somit in bestimmten (Aroma)Kompositionen, insbesondere solchen, die nicht in Richtung (Pfeffer)Minz-Aroma gehen, unerwünscht sein.

In J. Soc. Cosmet. Chem. 1978, 29, 185-200 wurden die Ergebnisse einer Studie an ca. 1200 Verbindungen präsentiert, bei der die Verbindungen L-Menthancarbonsäure-N-ethylamid ("WS3") und insbesondere *N*^{α}-(L-Menthancarbonyl)-glycinethylester ("WS5") als die stärksten Kühlsubstanzen gefunden wurden. Letzterer hat bei starker Wirkung aber den Nachteil, hydrolyseempfindlich zu sein und dabei die entsprechende freie Säure N^{α}-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlsubstanzen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten (z.B. die Menthancarbonsäure-N-(alkyloxyalkyl)amide nach JP 2004059474) oder sind zusätzlich stark reizend (WS5: N-[[5-Methyl-2-(1-methylethyl)cyclohexyl]-carbonyl]glycinethylester, US 2005/0222256).

N^{α}-(Menthancarbonyl)alkyloxyalkylamide wurden in JP 2004059474 beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar.

FR 2 577 922 offenbart die Herstellung von L-Menthyl-3-hydroxybutyrat und dessen Verwendung als Kühlsubstanz. Zur Herstellung dieser Verbindung wird die Ketogruppe des L-Menthyl-3-oxobutyrats (Verbindung der Formel (I-X), unten) reduziert. L-Menthyl-3-oxobutyrat wiederum wird gemäß FR 2 577 922 mittels Veresterung aus Menthol und Diketen erhalten; ein Hinweis auf die Kühlwirkung des L-Menthyl-3-oxobutyrats findet sich dort nicht. Die vorliegende Erfindung umfasst nicht die Reaktionsgemische gemäß FR 2 577 922.

Agric. Biol. Chem. 1983, 47, 1689-1690 offenbart die Synthese von (L)-Menthyl-3-oxohexanoat ((Verbindung der Formel (I) mit R1 bis R4 = H, R5 = Ethyl, unten) aus Ethylbromid und I-Menthylacetoacetat (Verbindung der Formel (1-X), unten). (L)-Menthyl-3-oxohexanoat wird als Synthesezwischenprodukt eingesetzt, es werden keinerlei Angaben über eine Kühlwirkung der offenbarten Verbindungen gemacht.

In DE 38 16 360 und DE 38 16 361 werden (-)-Menthylverbindungen der unten angegebenen Formel (I) mit R1 und R2 = H beschrieben. Diese Verbindungen werden als Ausgangsmaterialien zur Herstellung von pharmakologisch wirksamen 1,4-Dihydropyridindicarbonsäure-(-)-menthylestern eingesetzt. Eine Kühlwirkung der offenbarten Verbindungen ist nicht beschrieben.

Es war daher die primäre Aufgabe der vorliegenden Erfindung, Verbindungen bzw. Gemische von Verbindungen anzugeben, die eine starke physiologische Kühlwirkung besitzen, eine gute und im Vergleich zu bekannten Kühlwirkstoffen verbesserte (Hydrolyse-)Stabilität aufweisen und dabei in Nahrungs- und/oder Genussmitteln und/oder Mundpflegeprodukten und/oder oralen pharmazeutischen Zubereitungen und/oder kosmetischen Zubereitungen als Kühlsubstanzen (Kühlwirkstoffe) eingesetzt werden können. Die anzugebenden Verbindungen bzw. Verbindungsgemische sollten vorzugsweise einen möglichst schwachen Eigengeschmack zeigen, insbesondere wenig oder gar nicht bitter schmecken sowie möglichst nicht reizend sein.

Die primäre Aufgabe wird erfindungsgemäß gelöst durch die Verwendung einer Verbindung der Formel (I) oder (ent-I)
oder
einer Mischung bestehend aus zwei, drei oder mehr Verbindungen der Formel (I) oder (ent-I) 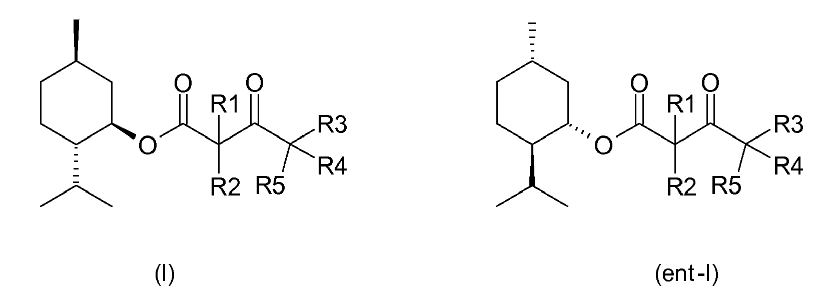
(a) als Kühlsubstanz für nicht-therapeutische und therapeutische Zwecke oder
(b) zur Herstellung eines Arzneimittels (insbesondere eines Arzneimittels mit physiologischer Kühlwirkung),
wobei in jeder der Formeln (I) und (ent-I) R1, R2, R3, R4 und R5 unabhängig voneinander jeweils Wasserstoff oder einen linearen, verzweigten oder cyclischen, gesättigten oder ungesättigten Kohlenwasserstoffrest (Rest, der ausschließlich C- und H-Atome umfasst) mit 1 bis 4 Kohlenstoffatomen bedeuten.

Die Verbindungen der Formeln (I) und (ent-I) werden nachfolgend auch als Menthyl-3-oxocarbonsäureester bezeichnet.

Innerhalb der Gruppe der erfindungsgemäß zu verwendenden Verbindungen der Formeln (I) oder (ent-I) bzw. den entsprechenden Mischungen sind bestimmte Verbindungen bevorzugt. Bevorzugt ist es insbesondere, wenn die Verbindung bzw. zumindest eine der Verbindungen in der Mischung ausgewählt ist aus der Gruppe bestehend aus Verbindungen der Formeln (I) und (ent-I), wobei R1, R2, R3, R4 und R5 unabhängig voneinander jeweils Wasserstoff oder einen Methyl-, Ethyl-, Propyl-, Cyclopropyl-, 2-Propyl-, 2-Propenyl-, 1-Propenyl-, 2-Methylpropyl-, Methyl-2-propenyl, Cyclobutyl-, 1-Butyl-, 2-Butyl-, tert.-Butyl- oder Cyclopropylmethylrest bedeuten.

Hierbei ist die Verbindung bzw. zumindest eine der Verbindungen in der Mischung vorzugsweise ausgewählt aus der Gruppe bestehend aus Verbindungen der Formeln (I) und (ent-I), wobei R1 und R2 Wasserstoff bedeuten. Sofern R1 und R2 Wasserstoff bedeuten, bedeuten R3, R4 und R5 vorzugsweise einen linearen oder verzweigten, vorzugsweise gesättigten Kohlenwasserstoffrest mit 1 bis 4 Kohlenstoffatomen, bevorzugt Methyl-, Ethyl-, Propyl-, Cyclopropyl-, 2-Propyl-, 2-Propenyl, 1-Propenyl-, 2-Methylpropyl-, Methyl-2-propenyl-, 1-Butyl-, 2-Butyl-, tert.-Butyl-, Cyclopropylmethyl-.

Vorzugsweise ist die (vorstehend vorzugsweise als bevorzugt bezeichnete) Verbindung bzw. zumindest eine der (vorstehend vorzugsweise als bevorzugt bezeichneten) Verbindungen in der Mischung ausgewählt aus der Gruppe bestehend aus Verbindungen der Formeln (I) und (ent-I), wobei R3 und R4 Wasserstoff bedeuten und R5 Wasserstoff oder Methyl bedeutet..

Besonders bevorzugt wird die Verbindung bzw. eine der Verbindungen in der Mischung ausgewählt aus der Gruppe bestehend aus Verbindungen der Formeln (I) und (ent-I), wobei R1, R2, R3 und R4 Wasserstoff bedeuten und R5 Wasserstoff oder Methyl bedeutet.

Ganz besonders bevorzugte Einzelverbindungen sind somit Verbindungen der Formeln (I) und (ent-I) mit R1, R2, R3, R4 = H und R5 = H oder CH₃, d. h.

L-Menthyl-3-oxobutyrat (I-X)
(R1, R2, R3, R4, R5 =H)

L-Menthyl-3-oxopentanoat (I-XX)
(R1, R2, R3, R4 = H; R5 = CH₃) 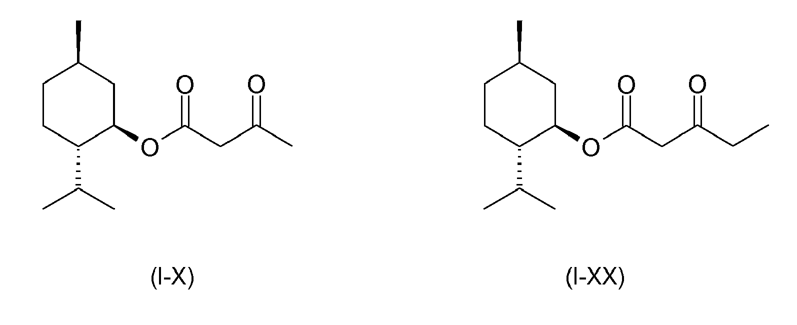 sowie die entsprechenden Verbindungen mit der Konfiguration gemäß Formel (ent-I).

Die genannten Einzelverbindungen liegen somit wahlweise in der Konfiguration gemäß Formel (I) oder (ent-I) vor; sie sind in der Literatur bislang noch nicht als Kühlsubstanzenbeschrieben worden.

Die erfindungsgemäß zu verwendenden Mischungen bestehen aus zwei, drei oder mehr Verbindungen der Formeln (I) oder (ent-I), vorzugsweise jeweils in einer der vorstehend als bevorzugt angegebenen Ausgestaltungen. Die Anwesenheit einer Verbindung der Formel (I) neben einer Verbindung der Formel (ent-I), wobei in den Formeln (I) bzw. (ent-I) die Bedeutung der jeweiligen Gruppen R1, R2, R3, R4 und R5 identisch ist, ist in manchen Fällen besonders bevorzugt; insbesondere bevorzugt ist die Anwesenheit von Enantiomerenpaaren. Des Weiteren ist bevorzugt ist es, wenn in einer erfindungsgemäß zu verwendenden Mischung nicht nur eine vorstehend als besonders bevorzugt bezeichnete Verbindung der Formel (I) oder (ent-I) vorhanden ist, d. h. nicht nur eine Verbindung, die eine, mehrere oder ausschließlich besonders bevorzugte Gruppen R1, R2 , R3, R4 und R5 umfasst, sondern zwei oder mehr als besonders bevorzugte erfindungsgemäße Verbindungen der Formeln (I) und/oder (ent-I). Vorzugsweise sind somit zwei, drei oder sämtliche Verbindungen in einer erfindungsgemäß zu verwendenden Mischung bestehend aus Verbindungen der Formel (I) oder (ent-I) aus der Gruppe der als bevorzugt bezeichneten Verbindungen ausgewählt.

Die vorstehenden Ausführungen gelten entsprechend für die nachfolgend angegebenen weiteren Aspekte der vorliegenden Erfindung. Ein weiterer Aspekt der vorliegenden Erfindung betrifft eine Mischung bestehend aus oder umfassend:
(a) eine erste Verbindung der Formel (I) oder (ent-I) wie vorstehend definiert, insbesondere in einer der vorstehend als bevorzugt bezeichneten Ausgestaltungen,
   sowie ein oder mehr Substanzen ausgewählt aus der Gruppe bestehend aus:
   - als weitere Komponente des Bestandteils (a) eine zweite Verbindung oder zwei oder mehr weitere Verbindungen der Formel (I) oder (ent-I) wie vorstehend definiert insbesondere in einer der vorstehend als bevorzugt bezeichneten Ausgestaltungen(z.B. eine Mischung aus einer ersten Verbindung der Formel (I), worin R1 bis R5 = H bedeutet und einer zweiten Verbindung der Formel (I), worin R1 bis R4 = H und R5 = Methyl bedeutet),
   - als Bestandteil (b) eine Verbindung oder ein Gemisch von zwei, drei oder mehr Verbindungen der Formeln (IIa), (IIb), (IIc), (ent-IIa), (ent-IIb), (ent-IIc) 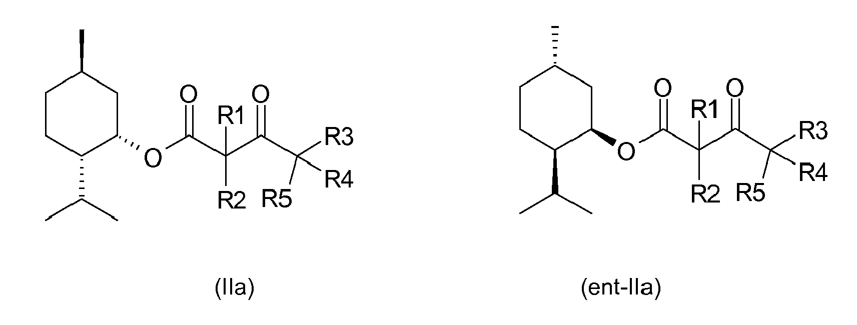 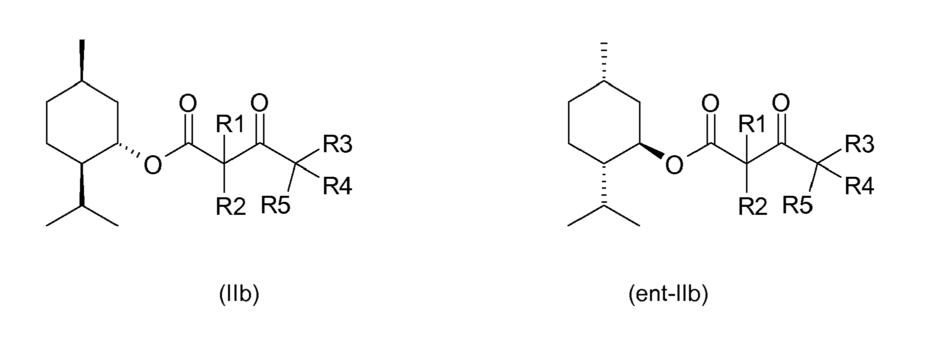 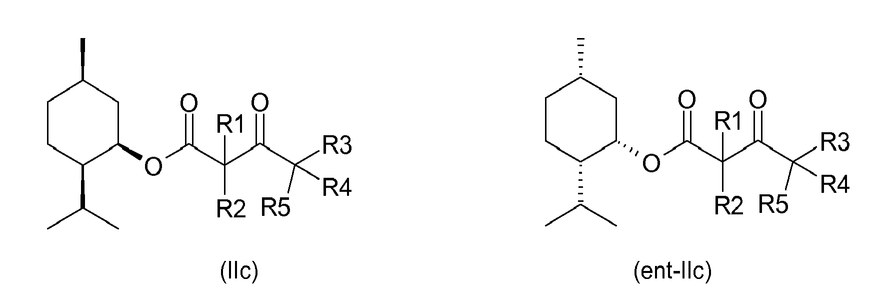
   wobei in jeder der Formeln (IIa), (IIb), (IIc), (ent-IIa), (ent-IIb) und (ent-IIc) R1, R2, R3, R4 und R5 jeweils unabhängig voneinander eine in den oben angegebenen Formeln (I) und (ent-I) angegebenen Bedeutungen besitzen,
   wobei die jeweiligen Bedeutungen von R1, R2, R3, R4 und R5 für die in der Mischung vorliegenden Verbindungen der Formeln (I), (ent-I), (IIa), (IIb), (IIc), (ent-IIa), (ent-IIb) und (ent-IIc) jeweils voneinander unabhängig sind,
   - als Bestandteil (c) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
   - als Bestandteil (d) einen oder mehrere Aromastoffe ohne physiologische Kühlwirkung. Aromastoffe sind im Rahmen des vorliegenden Textes vorzugsweise Stoffe, die aus natürlichen Grundstoffen erzeugt sind, insbesondere solche Stoffe, die in einem natürlichen verzehrbaren Material vorkommen und hierin eine aromagebende Wirkung haben (= natürliche Aromastoffe). Der Begriff "Aromastoffe" umfasst aber zudem auch Stoffe, die verzehrbaren Materialien zum Zwecke der Aromagebung zugesetzt werden (= naturidentische und/oder künstliche Aromastoffe).
   - als Bestandteil (e) einen oder mehrere trigeminal oder mundwässernd wirksame Stoffe ohne physiologische Kühlwirkung,
   wobei, sofern als erste Verbindung der Formel (I) L-Menthyl-3-oxobutyrat und als oder in Bestandteil (c) eine eine physiologische Kühlwirkung erzeugende Menge an L-Menthyl-3-hydroxybutyrat eingesetzt wird, die Mischung kein Reduktionsmittel für L-Menthyl-3-oxobutyrat umfasst.

Eine erfindungsgemäße Mischung liegt somit vor, wenn neben einer ersten Verbindung der Formel (I) oder (ent-I) wie vorstehend definiert, vorzugsweise in einer der vorstehend als bevorzugt bezeichneten Ausgestaltungen, als weitere Komponente eine zweite Verbindung oder zwei oder mehr weitere Verbindungen der Formel (I) oder (ent-I) wie vorstehend definiert, vorhanden sind.

Eine erfindungsgemäße Mischung liegt auch vor, wenn neben einer ersten Verbindung der Formel (I) oder (ent-I) wie vorstehend definiert als Bestandteil (b) eine Verbindung oder ein Gemisch von zwei, drei oder mehr Verbindungen der Formeln (IIa), (IIb), (IIc), (ent-IIa), (ent-IIb) und (ent-IIc) vorliegt. Häufig liegen hierbei gleichzeitig nicht nur eine erste Verbindung der Formel (I) oder (ent-I) neben einer oder mehreren Verbindungen der Formeln (IIa), (IIb), (IIc), (ent-IIa), (ent-IIb) und (ent-IIc) vor, sondern zumindest zwei Verbindungen der Formeln (I) bzw. (ent-I).

Eine erfindungsgemäße Mischung liegt ebenfalls vor, wenn neben zumindest einer ersten Verbindung der Formel (I) bzw. (ent-I) als Bestandteil (c) ein oder mehrere weitere Stoffe mit physiologischer Kühlwirkung vorliegen.

Entsprechendes gilt bei Anwesenheit eines oder mehrerer Aromastoffe ohne physiologische Kühlwirkung, die als Bestandteil (d) einer erfindungsgemäßen Mischung vorliegen können. Entsprechendes gilt ebenfalls, sofern als Bestandteil (e) ein oder mehrere trigeminal oder mundwässernd wirksame Stoffe ohne physiologische Kühlwirkung vorhanden sind. In jedem Falle liegt eine erfindungsgemäße Mischung vor. Die Bestandteile (b), (c), (d) und (e) liegen vorzugsweise in Kombination nebeneinander und neben dem Bestandteil (a) vor; bevorzugte Kombinationen sind nachfolgend angegeben.

Sofern als erste Verbindung der Formel (I) L-Menthyl-3-oxobutyrat und als oder in Bestandteil (c) eine eine physiologische Kühlwirkung erzeugende Menge an L-Menthyl-3-oxobutyrat eingesetzt wird, umfasst eine erfindungsgemäße Mischung vorzugsweise keine Reduktionsmittel für L-Menthyl-3-oxobutyrat. Das L-Menthyl-3-oxobutyrat wird somit nicht in der Mischung reduziert.

Die Verbindungen der Formeln (IIa), (IIb), (IIc), (ent-IIa), (ent-IIb) und (ent-IIc) fallen ebenso wie die Verbindungen der Formeln (I) und (la) unter die allgemeine Formel (III):

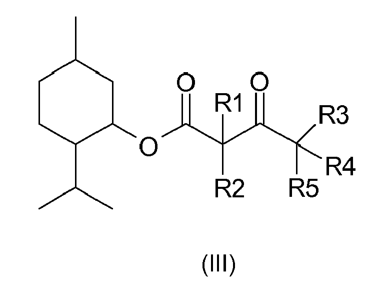

Wenn in Verbindungen des Typs (III) R1 und/oder R2 = H ist, können die Verbindungen - zumindest anteilig - auch in den entsprechenden tautomeren Enolformen vorliegen. Dies ist insbesondere abhängig von der Polarität , dem pH-Wert und der Temperatur des Mediums, in welches die Verbindungen gelöst oder eingearbeitet wurden.

Vorzugsweise umfasst eine erfindungsgemäße Mischung als oder in Bestandteil (b) eine Verbindung oder ein Gemisch von Verbindungen der Formeln (IIa), (IIb) und/oder (IIc).

Bevorzugt sind erfindungsgemäße Mischungen, die Bestandteil (a) sowie vorzugsweise (b) umfassen (sowie daneben gegebenenfalls noch Bestandteile (c), (d) und/oder (e)), wobei in einer, zwei, drei, mehr als drei oder sämtlichen in der Mischung enthaltenen Verbindungen (I), (ent-I), (IIa), (IIb), (IIc), (ent-IIa), (ent-IIb) und (ent-IIc) R1, R2, R3, R4 und R5 die vorstehend als für die Formeln (1) und (ent-I) bevorzugt angegebenen Bedeutungen besitzen.

In bevorzugten erfindungsgemäßen Mischungen, insbesondere in erfindungsgemäßen Mischungen, die als oder in Bestandteil (b) eine Verbindung oder ein Gemisch von Verbindungen der Formeln (IIa), (IIb) und/oder (IIc) umfassen, und weiter insbesondere in erfindungsgemäßen Mischungen, in denen sämtliche der unter die allgemeine Formel (III) fallenden Verbindungen Gruppen R1, R2 , R3, R4 und R5 umfassen, die vorstehend als bevorzugt bezeichnet sind, liegt das Gewichtsverhältnis von (a) der Gesamtheit von Verbindungen der Formel (I) und (ent-I) zu (b) der Gesamtheit von Verbindungen der Formeln (IIa), (IIb), (IIc), (ent-Ila), (ent-IIb) und (ent-IIc) im Bereich von 200 : 1 bis 4 : 1, bevorzugt im Bereich von 100 : 1 bis 10 : 1, insbesondere bevorzugt im Bereich von 100 : 1 bis 20 : 1.

Die Erfindung basiert auf der überraschenden Erkenntnis, dass die vorstehend angegebenen Menthyl-3-oxocarbonsäureester der Formeln (I) und (ent-I) sowie die vorstehend beschriebenen Mischungen, insbesondere die Verbindung der Formel (I) und die entsprechenden Mischungen, ein starkes und langanhaltendes Gefühl der Kälte auf der Haut oder Schleimhaut verursachen, insbesondere auf den Schleimhäuten des Mund-, Nasen- und Rachenraumes. Dabei zeigen die besagten Verbindungen keine anderen trigeminalen Effekte wie Schärfe, Tingling oder Betäubung und sind darüber hinaus nicht bitter. Gleichzeitig sind die erfindungsgemäß zu verwendenden Verbindungen der Formeln (I) und (ent-I) im Rahmen der üblichen wasserhaltigen Formulierungen und Zubereitungsbedingungen im Bereich von pH 1 bis pH 12, insbesondere im Bereich von pH 4 bis pH 9, hydrolysestabil, so dass sie in Formulierungen und Zubereitungen lange haltbar sind, so dass auch die jeweilige Formulierung bzw. Zubereitung selbst lange haltbar ist.

Um eine möglichst starke Kühlwirkung zu erhalten, wird in bevorzugten erfindungsgemäßen Mischungen, d. h. bei Kombinationen von zwei oder mehr Verbindungen der Formeln (I) und/oder (ent-I) oder bei Kombinationen von zumindest einer Verbindung der Formel (I) oder (ent-I) mit Verbindungen der Formeln (IIa), (IIb), (IIc), (ent-IIa), (ent-IIb) und (ent-IIc) der Anteil an Verbindungen der Formel (I) möglichst hoch gewählt, d. h. vorzugsweise größer oder gleich 90 Gew.-%, bevorzugt größer oder gleich 95 Gew.-%, bezogen auf das Gesamtgewicht sämtlicher in der Mischung enthaltener Verbindungen der Formeln (I), (ent-I), (IIa), (IIb), (IIc), (ent-IIa), (ent-IIb) und (ent-IIc) (Verbindungen der allgemeinen Formel (III). Eine erfindungsgemäße Mischung umfasst somit vorzugsweise eine oder mehrere Verbindungen der Formel (I), wobei der Anteil von Verbindungen der Formel (I) an dem Gesamtgewicht von Verbindungen der Formeln (I), (ent-I), (IIa), (IIb), (IIc), (ent-IIa), (ent-IIb) und (ent-IIc) zumindest 90 Gew.-% beträgt, vorzugsweise zumindest 95 Gew.-%.

Besonders bevorzugt sind erfindungsgemäße Mischungen, die neben dem Bestandteil (a) (sowie gegebenenfalls Bestandteilen (b), (d) oder (e)) als Bestandteil (c) einen oder mehrere weitere Stoffe mit physiologischer Kühlwirkung umfassen, wobei diese keinen geschmacklichen Effekt und keine Aromawirkung verursachen; vorzugsweise bewirken die Stoffe mit physiologischer Kühlwirkung somit lediglich eine (im Wesentlichen) "bloße" Kühlwirkung ohne weiteren sensorischen Effekt. Hierdurch wird vermieden, dass das Aromaprofil der Mischung beispielsweise in Richtung "Minze" (Pfefferminze) gebunden wird.

Ganz besonders bevorzugt sind erfindungsgemäße Mischungen bestehend aus oder umfassend einen Bestandteil (a) (sowie gegebenenfalls Bestandteilen (b), (c) oder (e)) sowie als Bestandteil (d) einen oder mehrere Aromastoffe ohne physiologische Kühlwirkung, wobei dieser Aromastoff bzw. diese Aromastoffe vorzugsweise einen Geschmackseindruck, einen geschmacksmodulierenden Effekt, einen trigeminalen Effekt und/oder einen mundwässernden Reiz verursacht bzw. verursachen. Es kann aber auch als Bestandteil (e) zusätzlich oder anstelle eines Bestandteiles (d) ein oder mehrere Stoffe vorgesehen sein, welche andere trigiminale Reize oder eine mundwässernde Wirkung verursachen. Die entsprechenden erfindungsgemäßen Mischungen besitzen eine angenehme Kühlwirkung und ein ausgeglichenes sensorisches Profil bei gleichzeitig hohem Impact, d. h. hohem geschmacklichem Ersteindruck.

Vorzugsweise umfasst eine erfindungsgemäße Mischung als Bestandteil (c) eine, zwei oder mehr Verbindungen ausgewählt aus der Gruppe bestehend aus: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, 1-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (z.B. Menthancarbonsäure-N-ethylamid [WS3], N^{α}-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (1-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydro-pyrimidin-2-one (z.B. Icilin oder verwandte Verbindungen, wie in WO 2004/026840 beschrieben).

Menthol (L-Menthol, D-Menthol, racemisches Menthol, Isomenthol, Neoisomenthol, Neomenthol), L-Menthyl-methylether, Menthylformiat, Menthylacetat,), Menthon, Isopulegol, I-(-)-Isopulegolacetat) und Cubebol haben hierbei einen geschmacklichen Effekt.

Der eine bzw. die mehreren weiteren Stoffe mit physiologischer Kühlwirkung, die als Bestandteil (c) in einer erfindungsgemäßen Mischung eingesetzt werden können, werden dabei vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (1-Menthoxy)-1,2-propandiol, (1-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernstein-säureesteramid), Menthancarbonsäureamide (z.B. Menthancarbonsäure-N-ethylamid [WS3], N^{α}-(Menthancarbonyl)glycinethylester [WS5], Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) .

Gemische aus einer Verbindung der Formel (I) mit R1, R2, R3, R4 und R5 = H und L-Menthyllactat zeigten überraschenderweise eine länger anhaltende Kühlwirkung als die Einzelkomponenten; die Verlängerung der Kühlwirkung beruht vermutlich auf einem Synergismus.

Der oder die mehreren weiteren Stoffe mit physiologischer Kühlwirkung, die als Bestandteil (c) einer erfindungsgemäßen Mischung eingesetzt werden können, sind vorzugsweise Stoffe, welche zumindest im Wesentlichen eine physiologische Kühlwirkung verursachen, ohne gleichzeitig eine geschmackliche Wirkung zu verursachen. Solche bevorzugten Stoffe sind: Menthylether (z.B. (I-Menthoxy)-1,2-propandiol, (I-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (z.B. Menthancarbonsäure-N-ethylamid [WS3], N^{α}-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Bestimmte Mischungen von Menthancarbonsäureamiden mit Kühlwirkstoffen wie acyclischen Carbonsäureamiden und L-Menthyllactat sowie gegebenenfalls weiteren Kühlwirkstofffen oder trigminalen Reizstoffen sind zum Beispiel in WO 2005/0117811 beschrieben; es findet sich dort jedoch kein Hinweis auf den Einsatz der erfindungsgemäßen Menthyl-3-oxocarbonsäureester (Verbindungen der Formeln (I) und (ent-I)).

Die Synthese der Verbindungen der Formel (I) bzw. die Synthese entsprechender Gemische wird vorzugsweise durch Umsetzung der entsprechenden 3-Oxoalkancarbonsäuremethyl- oder -ethylester oder eines anderen entsprechenden aktivierten 3-Oxoalkancarbonsäurederivats mit einem entsprechenden 2-Isopropyl-5-methyl-cyclohexanol in Substanz oder in einem aprotischen Lösungsmittel wie Aceton, Ether, Methylenchlorid, Tetrahydrofuran oder Toluol in Abwesenheit oder Gegenwart bekannter Lewis-Säuren wie Zinckchlorid, Zinn-IVchlorid, Eisen-III-chlorid oder in Gegenwart anderer Umesterungskatalysatoren wie Pyridin, Imidazol oder 4-Dimethylaminopyridin (DMAP) erreicht (für eine Übersicht literaturbekannter Methoden zur Synthese von 3-Oxoalkansäureestern siehe Eur. J. Org. Chem. 2000, 1633 und die dort zitierte Literatur).

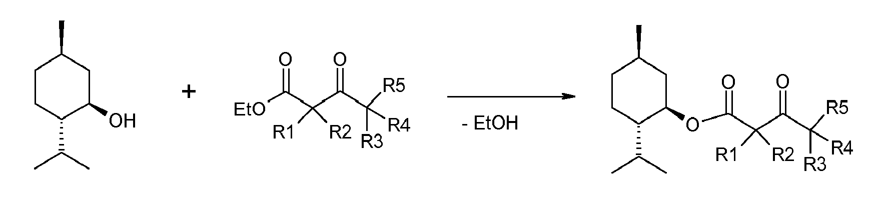

Die Synthese von Verbindung (I) mit R1 bis R5 = H wurde mehrfach in der Literatur beschrieben (H. Hennecka in E.Müller (Ed.) "Houben-Weyl, Methoden Der Organischen Chemie", Bd. 8, S.527, Georg Thieme Verlag, Stuttgart 1952; und dort zitierte Literatur).

Entsprechende Synthesewege sind bevorzugt für die Herstellung einer Verbindung der Formel (ent-1) sowie für die Herstellung von Verbindungen der Formeln (IIa), (IIb), (IIc), (ent-IIa), (ent-IIb), (ent-IIc).

Die Rohprodukte der Synthese werden vorzugsweise durch physikalische, gegebenenfalls auch enantioselektive oder enantiospezifische Trennmethoden, z.B. Extraktion, Verteilungsmethoden, Kristallisation, Destillation, Chromatographie, Sublimation, Wasserdampfdestillation, Umkehrosmose, Permeation oder dergleichen aufgereinigt bzw. angereichert, wobei die Trennmethode vorzugsweise so gewählt ist, dass die Menthyl-3-oxocarbonsäureester der Formel (I) oder (ent-I) oder deren Gemische nach der Trennoperation in einem Anteil von größer 90 Gew.-%, bevorzugt größer 95 Gew.-%, vorliegen, bezogen auf die Gesamtmenge an in dem aufgereinigten Produkt vorliegenden Verbindungen der Formeln (I), (ent-I), (IIa), (IIb), (IIc), (ent-IIa), (ent-IIb) und (ent-IIc) (d. h. Verbindungen der allgemeinen Formel (III).

Weiter oben wurden bereits bevorzugte erfindungsgemäße Mischungen offenbart, die neben einer Verbindung der Formel (I) oder (ent-I) oder einer Mischung solcher Verbindungen (als Bestandteil (a)) als Bestandteil (c) einen oder mehrere weitere Stoffe mit physiologischer Kühlwirkung umfassen. Es wurde in diesem Zusammenhang bereits ausgeführt, dass der weitere Stoff bzw. einer, mehrere oder sämtliche der weiteren Stoffe, die als Bestandteil (c) eingesetzt werden, vorzugsweise keinen geschmacklichen Effekt verursachen; bevorzugte entsprechende weitere Stoffe mit physiologischer Kühlwirkung wurden angegeben. Besonders bevorzugte erfindungsgemäße Mischungen enthalten 0,05 bis 90 Gew.-% des Bestandteils (a) sowie 0,01 bis 90 Gew.-% des Bestandteils (c), bezogen auf das Gesamtgewicht der Bestandteile (a), (b), (c), (d) und (e) (soweit vorhanden), wobei hinsichtlich bevorzugter Ausgestaltungen der Bestandteile (a) und (c) die jeweiligen obigen Ausführungen zu beachten sind. Neben den Bestandteilen (a) und (c) kann eine derartige bevorzugte Mischung noch einen oder mehrere der Bestandteile (b), (d) oder (e) umfassen, wie sie weiter oben angeben sind. Daneben kann eine bevorzugte erfindungsgemäße Mischung auch eine oder mehrere Komponenten aus der Gruppe umfassen, die besteht aus: Lösungsmittel, Trägerstoffe, sonstige Hilfsmittel (wie z. B. Farbstoffe, Konservierungsstoffe, Stabilisierungsmittel und/oder Verdickungsmittel). Besonders bevorzugt ist in erfindungsgemäßen Mischungen der Einsatz von einem oder mehreren Aromastoffen ohne physiologische Kühlwirkung (Bestandteil (d)), wobei diese Aromastoffe vorzugsweise neben ihrem eigentlichen geruchlichen Aromawert auch einen Geschmackseindruck, einen geschmacksmodulierenden Effekt oder einen trigeminalen, aber nicht-kühlenden Effekt und/oder einen mundwässernden Reiz verursachen. Sofern die besagten Aromastoffe einen trigeminalen oder mundwässernden Reiz verursachen, können diese gleichzeitig als Bestandteil (e) einer erfindungsgemäßen Mischung aufgefasst werden. Bevorzugt ist als oder in Bestandteil (d) der Einsatz von Aromastoffen, welche einen oder mehrere der nachstehenden bevorzugten Geschmackseindrücke, geschmacksmodulierenden Effekte oder trigminalen Reize verursachen:
bevorzugte Geschmackseindrücke: süß, Umami, bitter, salzig, sauer;
bevorzugte geschmacksmodulierende Effekte: bitter-maskierend, umami-verstärkend, süß-verstärkend, salz-verstärkend, sauer-maskierend;
bevorzugte trigeminale Reize: Schärfe, Wärme, Kribbeln, Stechen.

Besonders bevorzugt als/oder in Bestandteil (d) einer erfindungsgemäßen Mischung, d. h. als Aromastoffe ohne physiologische Kühlwirkung sind Pellitorine gemäß WO 2004/000787 bzw. US 2004/0241312 sowie Alkencarbonsäure-N-alkylamide gemäß DE 103 51 422.

Die Erfindung betrifft auch der Ernährung, der Mundhygiene oder dem Genuss dienende oder pharmazeutische oder kosmetische Zubereitungen, umfassend eine zum Erreichen einer physiologischen Kühlwirkung auf der Haut und/oder Schleimhaut ausreichende Menge einer Verbindung der Formel (1) oder (ent-I) oder einer Mischung bestehend aus zwei, drei oder mehr Verbindungen der Formel (I) oder (ent-I) wie vorstehend definiert, insbesondere in einer als bevorzugt bezeichneten Ausgestaltung. Insbesondere soll die eingesetzte Menge der Verbindung bzw. der erfindungsgemäßen Mischung zum Erreichen einer physiologischen Kühlwirkung auf der Schleimhaut im Mund-, Nasen- und/oder Rachenraum ausreichen.

Eine erfindungsgemäße Zubereitung umfasst oder besteht vorzugsweise aus einer erfindungsgemäßen Mischung. Sofern in dieser Mischung als erste Verbindung der Formel (I) L-Menthyl-3-oxobutyrat und als oder in Bestandteil (c) eine eine physiologische Kühlwirkung erzeugende Menge an L-Menthyl-3-hydroxybutyrat eingesetzt wird, umfasst die Zubereitung vorzugsweise kein Reduktionsmittel für L-Menthyl-3-oxobutyrat. Vorzugsweise umfasst die Zubereitung auch keineBestandteile, die L-Menthyl-3-oxobutyrat durch (bio-)chemische Reaktionen in L-Menthyl-3-hydroxybutyrat zu überführen vermögen.

Bevorzugte erfindungsgemäße Zubereitungen umfassen übliche Grund-, Hilfs- und Zusatzstoffe für der Ernährung, der Mundhygiene oder dem Genuss dienende oder pharmazeutische oder kosmetische Zubereitungen. Eine bevorzugte erfindungsgemäße Zubereitungen umfasst
- insgesamt 0,0001 Gew.-% bis 20 Gew.-%, bevorzugt 0,0001 bis 10 Gew.-%, besonders bevorzugt 0,001 Gew.-% bis 0,5 Gew.-% an Verbindungen der Formel (I), bezogen auf das Gesamtgewicht der Zubereitung,
- insgesamt 0,0000001 bis 99,99 Gew.-%, vorzugsweise 10 bis 80 Gew.-% an Verbindungen der Formel (ent-I), Bestandteilen (b), (c), (d) und/oder (e) wie vorstehend definiert, insbesondere in einer als bevorzugt bezeichneten Ausgestaltung, sowie weiteren Grund-, Hilfs- und Zusatzstoffen mit Ausnahme von Wasser, bezogen auf das Gesamtgewicht der Zubereitung,
- 0 bis 99,99 Gew.-% Wasser, bezogen auf das Gesamtgewicht der Zubereitung, vorzugsweise 5 bis 80 Gew.-%.

Bevorzugte der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Liköre, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nicht überzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundhygiene dienende Zubereitungen sind vorzugsweise Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi.

Zahnpflegemittel (als Basis für der Mundpflege dienende Zubereitungen), welche die erfindungsgemäße Verbindungen, Gemische oder Mischungen enthalten, umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Natrium-Cyclamat, Sucralose, Acesulfam-K oder ZuckeralkoholeGeschmackskorrigenzien für unangenehme Geschmackseindrücke wie z.B Hydroxyflavanone nach US 2002/0188019, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), die erfindungsgemäße Verbindungen, Gemische oder Mischungen enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugte erfindungsgemäße pharmazeutische Zubereitungen im Sinne der Erfindung sind orale Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Erfindungsgemäße kosmetische Zubereitungen können z. B. in einer der folgenden Formen vorliegen: Seife, Syndet, flüssiges Wasch-, Dusch-, oder Badepräparat, Emulsion (als Lösung, Dispersion, Suspension, Creme, Lotion oder Milch je nach Herstellungsverfahren und Inhaltsstoffen vom Typ "Wasser-in-Öl" (W/O), "Öl-in-Wasser" (O/W) oder multiple Emulsion, PIT Emulsion, Emulsionsschaum, Mikroemulsion, Nanoemulsion, Pickering Emulsion), Salbe, Paste, Gel (inklusive Hydro-, Hydrodispersions-, Oleogel), Öl, Toner, Balsam, Serum, Puder, Eau de Toilette, Toilette, Eau de Cologne, Perfum, Wachs, als Stift, Roll-On, (Pump-)Spray, Aerosol (schäumend, nicht schäumend oder nach-schäumend), Fusspflegemittel (inklusive Keratolytika, Desodorant), Bartreinigungs- oder -pflegemittel, Insekten abwehrendes Mittel, Sonnenschutzmittel, Aftersun-Präparat, Rasiermittel (z.B. Rasierschäume, -seifen oder -gele) oder After-Shave (Balm, Lotion), Haarentfernungsmittel, Haarpflegemittel wie z.B. Shampoo (z.B. 2-in-1 Shampoo, Anti-Schuppen-Shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (z.B. Gel oder Wachs), Blondiermittel, Haaraufheller, Haarconditioner, Haarmousse, Haartönung, Haarfärbemittel (z.B. temporäre, direktziehende, semipermanente, permanente Haarfärbemittel), Nagelpflegemittel wie z.B. Nagellack und Nagellackentferner, Deodorant und / oder Antitranspirant, Mundwasser, Munddusche, Make-Up, Make-Up-Entferner, Augenpflege, Lippenkosmetika, Lippenpflegemittel, dekorative Kosmetik (z.B. Puder, Lidschatten, Kajalstift, Lippenstift), Badeartikel (z.B. Kapsel), oder Maske.

Erfindungsgemäße Zubereitungen, die erfindungsgemäß zu verwendende Verbindungen oder eine erfindungsgemäße Mischung umfassen, werden vorzugsweise hergestellt, in den die Verbindung, das Gemisch oder die Mischung, z. B. eine Mischung, die neben einer erfindungsgemäß zu verwendenden Verbindung einen festen oder flüssigen Trägerstoff umfasst, in einer Basis-Zubereitung eingearbeitet wird. Vorteilhafterweise werden zunächst als Lösung vorliegende erfindungsgemäße Mischungen, die eine erfindungsgemäß zu verwendende Verbindung umfassen, durch Sprühtrocknung in eine feste Zubereitung überführt.

Erfindungsgemäße Zubereitungen können gemäß einer alternativen bevorzugten Ausführungsform hergestellt werden, indem die erfindungsgemäß zu verwendenden Verbindungen bzw. Mischungen, gegebenenfalls mit weiteren Bestandteilen der erfindungsgemäßen Zubereitung, zunächst in Emulsionen, in Liposomen, z. B. ausgehend von Phosphatidylcholin, in Microsphären, in Nanosphären oder auch in Kapseln, Granulaten oder Extrudaten aus einer für Lebens- und Genussmittel oder kosmetische Zubereitungen geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) oder nichtnatürlichen Matrixmaterialien (wie Polyharnstoff) eingearbeitet werden. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und ggfs. einer geeigneter Kombination der vorgenannten Verfahren behandelt werden.

In einem weiteren bevorzugten Herstellungsverfahren werden die erfindungsgemäß zu verwendenden Verbindungenbzw. die erfindungsgemäßen Mischungen zunächst mit einem oder mehreren geeigneten Komplexbildnern, beispielsweise mit Cyclodextrinen oder Cyclodextrinderivaten, bevorzugt alpha-, beta- oder gamma-Cyclodextrin, komplexiert und in dieser komplexierten Form eingesetzt.

Besonders bevorzugt ist eine erfindungsgemäße Zubereitung, bei der die Matrix so gewählt ist, dass die erfindungsgemäß zu verwendenden Verbindungen oder erfindungsgemäßen Mischungen, insbesondere Mischungen, die weitere Kühlwirkstoffe und/oder Aromen umfassen, verzögert von der Matrix freigegeben werden, so dass eine langanhaltende Kühlwirkung erreicht wird.

Als Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Ein weiterer Aspekt der vorliegenden Erfindung betrifft ein Verfahren zum Erreichen einer physiologischen Kühlwirkung auf der Haut und/oder Schleimhaut. Ein solches erfindungsgemäßes Verfahren kann zu therapeutischen oder nichttherapeutischen (z. B. kosmetischen) Zwecken durchgeführt werden und umfasst den folgenden Schritt:
- Applizieren einer zum Erreichen einer physiologischen Kühlwirkung ausreichenden Menge
   (i) einer Verbindung der Formel (I) oder (ent-I) oder einer Mischung bestehend aus zwei, drei oder mehr Verbindungen der Formel (I) oder (ent-I) (wie oben definiert, vorzugsweise in einer oben beschriebenen bevorzugten Ausgestaltung),
   (ii) einer erfindungsgemäßen Mischung (wie oben beschrieben, vorzugsweise in einer als bevorzugt beschriebenen Ausgestaltung) oder
   (iii) einer erfindungsgemäßen Zubereitung (wie oben beschrieben, vorzugsweise in einer als bevorzugt angegebenen Ausgestaltung),
   auf die Haut und/oder Schleimhaut.

Weitere Aspekte der vorliegenden Erfindung ergeben sich aus den nachfolgenden Beispielen sowie der beigefügten Patentansprüchen.

### Beispiele

Die Beispiele dienen nur zur Verdeutlichung der Erfindung, ohne diese damit einzuschränken. Sofern nicht anders angegeben, beziehen sich alle Angaben auf das Gewicht.

### Beispiel 1: Synthese von (L)-Menthyl-3-oxo-butyrat (I ; R1 bis R5 = H)

Ein Gemisch von 156 g (1 mol) (L)-Menthol und 130 g (1 mol) Acetessigsäureethylester wurde unter Rühren bis auf eine Temperatur von 140°C erhitzt und das bei der einsetzenden Reaktion freiwerdende Ethanol über eine 15 cm Vigreux-Kolonne mit angeschlossenem Kühler abdestilliert. Nach Beendigung des Abdestillierens des Ethanols (ca. 5 h) wurde der im Reaktionskolben verbliebene Rückstand über die angeschlossene Vigreux-Kollone im Vakuum fraktionierend destilliert. Bei 140 - 145°C / 10 mbar wurden 235 g (98 % d. Th.) (L)-Menthyl-3-oxo-butyrat als farbloses Öl , welches nach längerem Stehen bei Raumtemperatur zu farblosen Kristallen vom Schmp. 35 - 36°C erstarrte, erhalten.

### Beispiel 2 : Synthese von (L)-Menthyl-3-oxo-pentanoat (I ;R1 bis R4 = H; R5 = CH₃)

Ein Gemisch von 156 g (1 mol) (L)-Menthol und 142 g (1 mol) 3-Oxopentansäureethylester wurde unter Rühren bis auf eine Temperatur von 140°C erhitzt und das bei der einsetzenden Reaktion freiwerdende Ethanol über eine 15 cm Vigreux-Kolonne mit angeschlossenem Kühler abdestilliert. Nach Beendigung des Abdestillierens des Ethanols (ca. 5 h) wurde der im Reaktionskolben verbliebene Rückstand über die angeschlossene Vigreux-Kollone im Vakuum fraktionierend destilliert. Bei 145 - 152°C / 10 mbar wurden 248 g (98 % d. Th.) (L)-Menthyl-3-oxo-pentanoat als farbloses Öl erhalten.
1 H-NMR (CDCl3): ö = 0,77 (d, j = 6.9 Hz, 3H, H-9), 0,89 (d, j = 7.0 Hz, 3H, H-10), 0,91 (d, j = 6.6 Hz, 3H, H-7), 0,99 (m, 2H, H-6), 1,09 (t, j = 7.3 Hz,3H, H-13), 1,38 (m, 2H, H-2), 1,49 (m, 1 H , H-1), 1,68 (m, 2H, H-5), 1,87 (m, 1 H, H-8), 2,03 (m, 1 H, H-4), 2,56 (q, j = 7.3 Hz, 2H, H-12), 3,42 (s, 2H, H-11), 4,73 ppm (m, 1 H, H-3).

Tetramethylsilan (TMS) wurde als interner Standard benutzt.

### Anwendungsbeispiel 1: Kühlwirkung

Die Verbindungen aus Beispiel 1 und 2 wurden auf ihre sensorischen Eigenschaften, insbesondere ihre Kühlwirkung getestet. Dafür wurden sie jeweils in einer bestimmten Endkonzentration in einer aus Sucrose (Saccharose) und Wasser bereiteten Masse (Konditorenfondant, Lieferant Nordzucker AG, Nordstemmen) gelöst und in einer Runde von Experten bewertet. Die sensorischen Eindrücke wurden notiert und die Kühlwirkung anhand einer Skala von 1 (keine Kühlwirkung) bis 9 (extrem starke Kühlwirkung) bewertet.

Profil des L-Menthyl-3-oxobutyrates (Beispiel 1) bei einer Konzentration von 0,05 Gew.-%, bezogen auf die Gesamtzubereitung: schwach blumig, Kühlwirkung 5 - 6, nicht bitter.

Profil des L-Menthyl-3-oxopentanoates (Beispiel 2) bei einer Konzentration von 0,05 Gew.-%, bezogen auf die Gesamtzubereitung: Kühlwirkung 5 -6; verzögert einsetzend aber länger anhaltend als Beispiel 1, nicht bitter. Bezüglich der hautkühlenden Eigenschaften zeigte sich (Anwendungsort: Unterarme der Testpersonen), dass die erfindungsgemäßen Verbindungen, insbesondere L-Menthyl-3-oxobutyrat (I-X) und L-Menthyl-3-oxopentanoat (I-XX), im zeitlichen Verlauf ein ähnliches Kühlprofil aufweisen wie der häufig eingesetzte Kühlwirkstoff L-Menthyllactat (Frescolat ML®, Symrise GmbH & Co. KG), wobei insgesamt eine etwas schwächere Kühlintensität als bei dieser Vergleichssubstanz beobachtet wird.

### Anwendungsbeispiel 2: Aromamischung zum Erreichen einer Kühlwirkung

| **Inhaltsstoff** | **Anteil in %** |
|---|---|
| (L)-Menthyl-3-oxobutyrat (Beispiel 1) | 25 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischender Komponenten wird eine stark kühlende, ansonsten jedoch nahezu geschmacks- und geruchslose, bei Raumtemperatur (20 °C) flüssige Aromamischung erhalten.

### Anwendungsbeispiel 3: Aromamischung zum Erreichen einer Kühlwirkung

| **Inhaltsstoff** | **Anteil in %** |
|---|---|
| (L)-Menthyl-3-oxopentanoat (Beispiel 2) | 7,5 |
| L-Menthancarbonsäure-N-ethylamid (WS 3, z.B. Millenium) | 5 |
| L-Menthyllactat (Frescolat ML, Symrise) | 32,5 |
| O-L-Menthyl-O'-(2-hydroxyethyl)carbonat (Frescolat MGC, Symrise) | 5 |
| Propylenglycol | 50 |

Durch Mischen der Komponenten wird eine stark kühlende, ansonsten jedoch nahezu geschmacks- und geruchslose, bei Raumtemperatur (20 °C) flüssige Aromamischung erhalten.

### Anwendungsbeispiel 4: Aromamischung zum Erreichen einer Aroma- und Kühlwirkung

| **Inhaltsstoff** | **Anteil in %** |
|---|---|
| (L)-Menthyl-3-oxobutyrat (Beispiel 1) | 15 |
| Pfefferminzöl | 10 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischen der Komponenten wird eine stark kühlende, stark nach Pfefferminz riechende Aromamischung erhalten.

### Anwendungsbeispiel 5: Aromamischung zum Erreichen einer Kühlwirkung mit gleichzeitigem Tingling-Effekt

| **Inhaltsstoff** | **Anteil in %** |
|---|---|
| (L)-Menthyl-3-oxobutyrat (Beispiel 1) | 15 |
| Lösung aus 10 Gew.-% Pellitorin in Propylen-glycol/Pfefferminzöl | 10 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischender Komponenten wird eine stark kühlende Aromamischung erhalten, welche speichelanregend ist und einen Tingling-Effekt verursacht.

### Anwendungsbeispiel 6: Verwendung in Form einer Aromamischung in einer Zahnpasta

| **Teil** | **Inhaltsstoff** | **Einsatz in Gew.-%** |
|---|---|---|
| A | demineralisiertes Wasser | 22,00 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aromamischung aus Anwendungsbeispiel 2 | 1 |

Die Inhaltsstoffe der Teile A und B wurden jeweils für sich vorgemischt und zusammen unter Vakuum bei 25 - 30°C jeweils 30 min lang gut verrührt. Teil C wurde vorgemischt und zu A und B gegeben; D wurde hinzugefügt und die Mischung unter Vakuum bei 25 - 30°C weitere 30 min gut verrührt. Nach Entspannung war die Zahnpasta fertig und konnte abgefüllt werden.

### Anwendungsbeispiel 7: Verwendung als Kühlwirkstoff in einem zuckerfreien Kaugummi

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Spearmint-Pfefferminz-Eucalyptus-Aroma, enthaltend 5 Gew.-% (L)-Menthyl-3-oxobutyrat (Beispiel 1) | 1 |

Teile A bis D wurden gemischt und intensiv geknetet. Die Rohmasse wurde z.B. in Form von dünnen Streifen zu verzehrsfertigen Kaugummis verarbeitet.

### Anwendungsbeispiel 8: Verwendung als Kühlwirkstoff in einem Mundwasser

| **Teil** | **Inhaltsstoff** | **Einsatz in Gew.-%** |
|---|---|---|
| A | Ethanol | 10,00 |
| | Cremophor^{®} CO 40 (BASF, Detergenz) | 1,00 |
| | Benzoesäure | 0,12 |
| | Pfefferminz-Zitronenmelisse-Aroma, enthaltend 0,4 Gew.-% Pellitorin und 10 Gew.-% (L)-Menthyl-3-oxobutyrat (Beispiel 1) | 0,25 |
| B | demineralisiertes Wasser | 83,46 |
| | Sorbitol, 70% | 5,00 |
| | Natriumsaccharin 450 | 0,07 |
| | L-Blue 5000 e.c., 1% in Wasser (Farbstoff) | 0,10 |

Die Inhaltsstoffe der Teile A und B wurden jeweils für sich gemischt. Teil B wurde langsam in Teil A eingerührt, bis die Mischung homogen war.

### Anwendungsbeispiel 9: Halsbonbons mit flüssig-viskoser Kernfüllunq (centre-filled hard candy)

| | I (Gew.-%) | II (Gew.-%) |
|---|---|---|
| **Mischung A (Hülle) (80% der Bonbons)** | | |
| Zucker (Saccharose) | 58,12 | 49,37 |
| Glucosesirup (Feststoffgehalt 80%) | 41,51 | 49,37 |
| Aromamischung aus Anwendungsbeispiel 5 | 0,17 | 0,25 |
| 1-Menthol | 0,10 | - |
| Citronenöl | 0,10 | 0,10 |
| Citronensäure | - | 0,91 |
| Total: | 100 | 100 |

| **Mischung B (Kern) (20% der Bonbons)** | | |
|---|---|---|
| High Fructose Maissirup (Gehalt an festen Zuckern 85%, knapp 15% Wasser) | 84,38 | 84,36 |
| Glycerin | 15,0 | 15,0 |
| Lecithin | 0,02 | 0,02 |
| Zimtöl | - | 0,32 |
| Spearmintöl | 0,28 | - |
| Capsaicin | 0,05 | - |
| Vanillylalkohol-n-butylether | - | 0,10 |
| Roter Farbstoff, als 5%ige wässrige Lösung | 0,20 | 0,20 |
| Vanillin | 0,07 | - |
| Total | 100 | 100 |

In Anlehnung an die in US 6,432,441 (dort Beispiel 1) sowie die in US 5,458,894 bzw. US 5,002,791 beschriebenen Verfahren wurden Bonbons mit flüssigviskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Anwendungsbeispiel 10: Kaugummi

Die Kaugummibase K2 bestand aus folgenden Zutaten: 28,5% Terpenharz, 33,9% Polyvinylacetat (MW = 14,000), 16,25% hydriertes Pflanzenöl, 5,5% Mono- und Diglyceride, 0,5% Polyisobuten (MW 75,000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis erfolgte analog zu US 6,986,907.

| | I (Gew.-%) | II (Gew.-%) | III (Gew.-%) |
|---|---|---|---|
| Kaugummibase K2 | 25,30 | 27,30 | 26,30 |
| Sorbitol | 61,48 | 59,48 | 61,80 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet | 0,50 | - | - |
| Kirscharoma, sprühgetrocknet | - | 1,20 | - |
| Aromamischung aus Anwendungsbeispiel 4, sprühgetrocknet | 1,50 | 1,80 | - |
| Aromamischung aus Anwendungsbeispiel 3 | 1,00 | - | 1,68 |

Die Kaugummis der Rezeptur (I) und (II) wurden als Streifen, die der Rezeptur (III) als Pellets ausgeformt.

### Anwendungsbeispiel 11: Gelatinekapsel zum Direktverzehr

| | I (Gew.-%) | II (Gew.-%) | III (Gew.-%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |
| Kernzusammensetzung: | | | |
| Pflanzenöl-Triglycerid (Kokosöl - Fraktion) | 79,39 | 68,40 | 58,25 |
| Zimt-Anis-Aroma | 10,00 | 20,90 | - |
| Eucalyptus-Aroma | - | - | 29,95 |
| Neotam und Aspartam | 0,01 | 0,05 | - |
| Sucralose | 0,22 | 0,30 | 0,70 |
| Aromamischung aus Anwendungsbeispiel 5 | 0,33 | - | - |
| Aromamischung aus Anwendungsbeispiel 3 | | 0,20 | 0,60 |
| (L)-Menthyl-3-oxobutyrat (Beispiel 1) | - | 0,05 | - |
| (-)-Menthonglycerinacetal (Frescolat MGA) | - | 0,10 | 0,40 |
| Vanillin | 0,05 | - | 0,10 |

Die zum Direktverzehr geeigneten Gelatinekapseln I, II, III wurden gemäß WO 2004/050069 hergestellt und hatten jeweils einen Durchmesser von 5 mm, das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.

### Anwendungsbeispiel 12: Kaubonbon

| | | |
|---|---|---|
| Wasser | | 7,80 % |
| Zucker | Raffinade C4 | 42,10 % |
| Glucose Sirup | Dextrose 40 | 37,30 % |
| gehärtetes Pflanzenfett | Schmelzpunkt 32-36°C | 6,60 % |
| Lecithin | Emulgator (Sojalecithin) | 0,30 % |
| Gelatine | Schweinegelatine | 0,80 % |
| Fondant | Typ - S30 | 4,80 % |
| Himbeeraroma | | 0,22 % |
| Aromamischung aus Anwendungs-beispiel 3 | | 0,08 % |

### Herstellungshinweise:

a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Aroma aus Beispiel 2 und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.

Beim Verzehr der Kaubonbons wird während des Kauens ein frischer, kühlender Himbeergeschmack wahrgenommen.

### Anwendungsbeispiel 13: Extrudat

| | | |
|---|---|---|
| Glukosesirup, sprühgetrocknet (DE-Wert: 31-34) | Glucidex 1T33W (Firma Roquette) | 62,0 % |
| Maltodextrin (DE-Wert: 17-20) | (Firma Cerestar) | 28,4% |
| Emulgator Monomuls | Emulgator auf der Basis von gehärtetem Palmöl; Schmelzpunkt: 64°C, (Firma Grünau) | 1,8% |
| Dextrosemonohydrat (DE-Wert: 99,5) | Dextrose, kristallwasserhaltig (Firma Cerestar) | 1,8 % |
| Wasser | | 2,0 % |
| Orangen-Vanillearoma | | 3,2 % |
| Aromamischung aus Anwendungsbeispiel 4 | | 0,8 % |

### Herstellungshinweis (siehe auch WO 03/092412):

Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

### Anwendungsbeispiel 14: Wirbelschichtgranulate

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aromamischung aus Anwendungsbeispiel 4, 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Anwendungsbeispiel 15: Teebeutel mit Rooibos bzw. schwarzem Tee und Extrudaten aus Auswendungsbeispiel 13 bzw. Granulaten aus Anwendungsbeispiel 14

Jeweils 800 g Rotbuschtee (Rooibos-Tee) wurden einmal mit 33 g der Extrudate aus Anwendungsbeispiel 13 und einmal mit 30 g Granulaten aus Anwendungsbeispiel 14 gemischt, portioniert und anschließend in Teebeutel abgefüllt.

Jeweils 800 g schwarzer Tee (Blattgrad Fannings) wurden einmal mit 33 g der Extrudate aus Anwendungsbeispiel 13 und einmal mit 30 g Granulaten aus Anwendungsbeispiel 14 gemischt, portioniert und anschließend in Teebeutel abgefüllt.

### Beispiele 16 - 22: Kosmetische Formulierungen

**16 = O/W Tagescreme**
**17 = O/W Hautlotion mit Pflanzenextrakt**
**18 = After-sun Balsam**
**19 = Körperspray für empfindliche Haut**
**20 = Sonnenschutz-Lotion (O/W) mit Breitbandschutz**
**21 = W/O Nachtcreme**
**22 = Shampoo**

| **MATERIAL (LIEFERANT)** | **INCI** | **GEW.-%** | | | | | | |
|---|---|---|---|---|---|---|---|---|
| | | **16** | **17** | **18** | **19** | **20** | **21** | **22** |
| -(-alpha-)-Bisabolol, natürlich (Symrise) | Bisabolol | | | 0,1 | | | 0,1 | |
| Abil 350 (Degussa-Goldschmidt) | Dimethicone | 0,5 | 2,0 | 1,0 | | | | |
| Allantoin (Merck) | Allantoin | | | 0,1 | | | | |
| Aloe vera Gel concentrate 10/1 (Symrise) | Water (aqua), Aloe barbadensis leaf juice | | | 3,0 | | | 3,0 | |
| Alugel 34 TH (Baerlocher) | Aluminium stearate | | | | | | 1,0 | |
| Symatrix (Symrise) | Maltodextrin, Rubus fructicosus (blackberry) leaf extract | 0,3 | 0,1 | 1,0 | 0,1 | 0,3 | | |
| Butylenglycol | Butylene glycol | | | 5,0 | | | | |
| Carbopol ETD 2050 (Noveon) | Carbomer | | | | | 0,2 | | |
| Carbopol Ultrez-10 (Noveon) | Carbomer | | 0,1 | | | | | |
| Cetiol OE (Cognis) | Dicaprylyl ether | | | 4,0 | | | | |
| Cetiol SB 45 (Cognis) | Butyrospermum parkii (shea butter) | | | 1,0 | | | | |
| Citronensäure, 10 %ige Lösung | Citric acid | | | | | | | 0,3 |
| Comperlan 100 (Cognis) | Cocamide MEA | | | | | | | 0,5 |
| Dihydroavenanthramid D (Symrise) | | 0,05 | 0,05 | 0,05 | 0,05 | 0,05 | 0,05 | 0,05 |
| Dow Corning 246 Fluid (Dow Corning) | Cyclohexasiloxane (and) cyclopentasiloxane | | | | | 2,0 | | |
| Dow Corning 345 Fluid (Dow Corning) | Cyclomethicone | | | | 0,5 | | | |
| D-Panthenol (BASF) | Panthenol | | | 1,0 | | | | |
| Dracorin CE (Symrise) | Glyceryl stearate citrate | 5,0 | | | | | | |
| Dracorin GMS (Symrise) | Glyceryl stearate | | 2,0 | | | | | |
| Dracorin GOC (Symrise) | Glyceryl oleate citrate, caprylic/capric triglyceride | | | | 2,0 | | | |
| Drago-Beta-Glucan (Symrise) | Water (aqua), butylene glycol, glycerol, Avena sativa (oat) kernel extract | 0,3 | | | | | | |
| Dragocid Liquid (Symrise) | Phenoxyethanol, methylparaben, ethylparaben, butylparaben, propylparaben, isobutylparaben | | 0,80 | 0,70 | | 0,70 | 0,80 | |
| Dragoderm (Symrise) | Glycerol, Triticum vulgare (wheat) gluten, water (aqua) | | | | | | | 2,0 |
| Drago-Oat-Active (Symrise) | Water (aqua), butylene glycol, Avena sativa (oat) kernel extract | | | | 1,0 | | | |
| Dragosan W/O flüssig (Symrise) | Polyglyceryl 3-polyricinoleate, sorbitan isostearate | | | | | | 1,0 | |
| Dragosan W/O P (Symrise) | Sorbitan isostearate, hydrogenated castor oil, ceresin, beeswax (Cera alba) | | | | | | 6,0 | |
| Dragosantol (Symrise) | Bisabolol | | | | 0,1 | 0,1 | | |
| Dragoxat EH (Symrise) | Ethylhexyl ethylhexanoate | 3,0 | 3,0 | | 4,0 | | | |
| EDETA B Pulver (BASF) | Tetrasodium EDTA | | | | | | | 0,1 |
| EDETA DB (BASF) | Disodium EDTA | | | | | 0,1 | | |
| Emulsiphos (Symrise) | Potassium cetyl phosphate, hydrogenated palm glycerides | | 2,0 | | | 1,5 | | |
| Ethanol, 96 %ig | Ethanol | | | | | | | |
| Extrapone Grüntee (Symrise) | Glycerol, water (aqua), Camellia sinensis leaf extract | | 0,2 | | | | | |
| Extrapone Hamamelis Destillat, farblos (Symrise) | Propylene glycol, Hamamelis virginiana (witch hazel), water (aqua), Hamamelis virginiana (witch hazel) extract | | | | | | 1,0 | |
| Extrapone Kamille (Symrise) | Glycerol, water (aqua), Chamomilla recutita (matricaria) flower extract | | 0,5 | | | | | |
| Extrapone Rosemarin GW (Symrise) | Glycerol, water (aqua), Rosmarinus officinalis (rosemary) leaf extract | | 0,3 | | | | | |
| L-Menthyl-3-oxobutyrat | | 0,4 | 0,3 | 0,4 | | 0,5 | 0,4 | 0,5 |
| L-Menthyl-3-oxopentanoat | | | 0,3 | | 0,5 | | 0,1 | |
| Frescolat ML, kristallin, (Symrise) | Menthyl lactate | 0,1 | | | | | | 0,5 |
| Genapol LRO Liquid (Clariant) | Sodium laureth sulphate | | | | | | | 37,0 |
| Glycerin, 85%ig in Wasser | Glycerol | 3,0 | 2,0 | 4,0 | | 4,7 | 2,0 | |
| Harnstoff | Urea | | | 0,5 | | | 1,0 | |
| Hydrolite-5 (1,2-Pentandiol) (Symrise) | Pentylene glycol | 1,0 | 0,5 | | 3,0 | 3,0 | | |
| Hydroviton (Symrise) | Water, glycerol, sodium lactate, TEA lactate, serine, lactic acid, urea, sorbitol, sodium chloride, lauryl diethylenediaminoglycine, lauryl aminopropylglycine, allantoin | 0,5 | 1,0 | | 1,0 | 1,0 | | 1,0 |
| Isodragol (Symrise) | Trüsononanoin | | 2,0 | | | | | |
| Isopropylpalmitat (Symrise) | Isopropyl palmitate | 4,0 | | | | | | |
| Karion F (Merck) | Sorbitol | | | | | | 2,0 | |
| Keltrol RD (CP-Kelco) | Xanthan gum | 0,2 | 0,1 | | | | | |
| Keltrol T (Danby-Chemie) | Xanthan gum | | | | | 0,2 | | |
| Lanette 16 (Cognis) | Cetyl alcohol | 1,0 | | | | | | |
| Lanette O (Cognis) | Cetearyl alcohol | | 3,0 | | | 1,0 | | |
| Lara Care A-200 (Rahn) | Galactoarabinan | | | 0,3 | | | | |
| Magnesiumsulfat (Merck) | Magnesium sulphate | | | | | | 0,7 | |
| L-Menthol (Symrise) | Menthol | | | 0,2 | | | | |
| Merquat 550 (Ondeo Nalco) | Polyquaternium-7 | | | | | | | 0,5 |
| Natriumbenzoat | Sodium benzoate | | | | | | | 0,5 |
| Neo Heliopan 357 (Symrise) | Butyl methoxydibenzoylmethane | | | | | 1,0 | | |
| Neo Heliopan AP (Symrise) | Disodium phenyl dibenzimidazole tetrasulphonate | | | | | 4,6 | | |
| Neo Heliopan AV (Symrise) | Ethylhexyl methoxycinnamate | | | | | 3,0 | | |
| Neo Heliopan Hydro (Symrise) | Phenylbenzimidazole sulphonic acid | | | | | 6,7 | | |
| Neo Heliopan MBC (Symrise) | 4-Methylbenzylidene camphor | | | | | 1,5 | | |
| Neo Heliopan OS (Symrise) | Ethylhexyl salicylate | | | | | 5,0 | | |
| Neutralöl | Caprylic/capric triglyceride | 6,0 | | | 4,0 | 2,0 | | |
| Oxynex 2004 (Merck) | BHT | | | | | | 0,1 | |
| Paraffinöl 5 grade E (Parafluid) | Paraffinum liquidum | | | | 4,0 | | | |
| PCL Liquid 100 (Symrise) | Cetearyl ethylhexanoate | 3,0 | 5,0 | | 7,0 | | | |
| PCL Solid (Symrise) | Stearyl heptanoate, stearyl caprylate | | 2,0 | | | | | |
| PCL-Liquid (Symrise) | Cetearyl ethylhexanoate, isopropyl myristate | | | | | | 12,0 | |
| Pemulen TR-2 (Noveon) | Acrylates/C10-30 alkyl acrylate crosspolymer | | | 0,3 | 0,2 | | | |
| 1,2-Propylenglycol | Propylene glycol | | 5,0 | | | | | |
| Sepigel 305 | Polyacrylamide, C13-14 isoparaffin, laureth-7 | | | | | | | |
| Natriumchlorid | Sodium chloride | | | | | | | 1,0 |
| Natriumhydroxid, 10 %ige Lösung | Sodium hydroxide | | 0,3 | 0,6 | 0,4 | | | |
| Sonnenblumenöl (Wagner) | Helianthus annuus (sunflower) seed oil | | | | | | 5,0 | |
| Mandelöl (Wagner) | Prunus dulcis | | | | | | 5,0 | |
| Symdiol 68 (Symrise) | 1,2-Hexanediol, caprylyl glycol | 0,5 | | | | | | |
| Parfüm (Symrise) | Fragrance | 0,3 | 0,3 | 0,3 | 0,2 | 0,4 | 0,4 | 0,5 |
| Tego Betaine L7 (Degussa) | Cocamidopropyl betaine | | | | | | | 6,0 |
| Tegosoft TN (Degussa) | C12-15 alkyl benzoate | | | 5,0 | | 5,0 | | |
| Triethanolamin | Triethanolamine | | | | | 0,5 | | |
| Retinylpalmitat in Öl (DSM Nutritional Products) | Retinyl palmitate | | | | | | 0,2 | |
| Tocopherolacetat (DSM Nutritional Products) | Tocopheryl acetate | | | 0,5 | | 0,5 | 3,0 | |
| Wasser, demineralisiert | Water (aqua) | ad 100 | ad 100 | ad 100 | ad 100 | ad 100 | ad 100 | ad 100 |

## Patentansprüche

1. Verwendung
einer Verbindung der Formel (I) oder (ent-I)
oder
einer Mischung bestehend aus zwei, drei oder mehr Verbindungen der Formel (I) oder (ent-I) 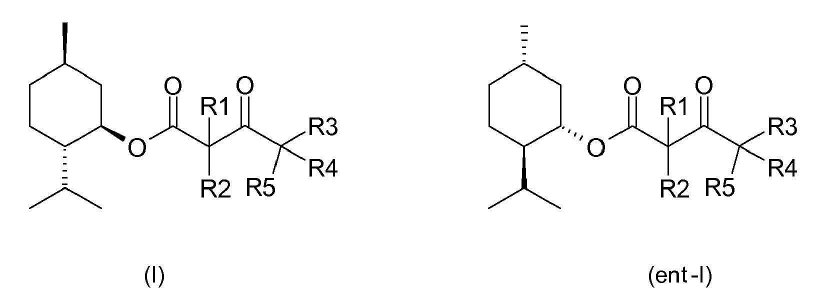
(a) als Kühlsubstanz für nicht-therapeutische Zwecke oder
(b) zur Herstellung eines Arzneimittels,
wobei in jeder der Formeln (I) und (ent-I)
R1, R2, R3, R4 und R5 unabhängig voneinander jeweils Wasserstoff oder einen linearen, verzweigten oder cyclischen, gesättigten oder ungesättigten Kohlenwasserstoffrest mit 1 bis 4 Kohlenstoffatomen bedeuten.

2. Verwendung nach Anspruch 1, wobei die Verbindung bzw. zumindest eine der Verbindungen in der Mischung ausgewählt ist aus der Gruppe bestehend aus Verbindungen der Formeln (I) und (ent-I),
wobei R1, R2, R3, R4 und R5 unabhängig voneinander jeweils Wasserstoff oder einen Methyl-, Ethyl-, Propyl-, Cyclopropyl-, 2-Propyl-, 2-Propenyl-, 1-Propenyl-, 2-Methylpropyl-, Methyl-2-propenyl, Cyclobutyl-, 1-Butyl-, 2-Butyl-, tert.-Butyl- oder Cyclopropylmethylrest bedeuten.

3. Verwendung nach einem der vorangehenden Ansprüche, wobei die Verbindung bzw. zumindest eine der Verbindungen in der Mischung ausgewählt ist aus der Gruppe bestehend aus Verbindungen der Formeln (I) und (ent-I), wobei R1 und R2 Wasserstoff bedeuten.

4. Verwendung nach einem der vorangehenden Ansprüche, wobei die Verbindung bzw. zumindest eine der Verbindungen in der Mischung ausgewählt ist aus der Gruppe bestehend aus Verbindungen der Formeln (I) und (ent-I),
wobei R3 und R4 Wasserstoff bedeuten und
R5 Wasserstoff oder Methyl bedeutet.

5. Verwendung nach einem der vorangehenden Ansprüche, wobei die Verbindung bzw. zumindest eine der Verbindungen in der Mischung ausgewählt ist aus der Gruppe bestehend aus Verbindungen der Formeln (I) und (ent-I), wobei R1, R2, R3 und R4 Wasserstoff bedeuten und R5 Wasserstoff oder Methyl bedeutet.

6. Mischung bestehend aus oder umfassend:
(a) eine erste Verbindung der Formel (I) oder (ent-I) wie in einem der vorangehenden Ansprüche definiert
sowie ein oder mehr Substanzen ausgewählt aus der Gruppe bestehend aus:
- als weitere Komponente des Bestandteils (a) eine zweite Verbindung oder zwei oder mehr weitere Verbindungen der Formel (I) oder (ent-I) wie in einem der vorangehenden Ansprüche definiert, - als Bestandteil (b) eine Verbindung oder ein Gemisch von zwei, drei oder mehr Verbindungen der Formeln (IIa), (IIb), (IIc), (ent-IIa), (ent-IIb), (ent-IIc) 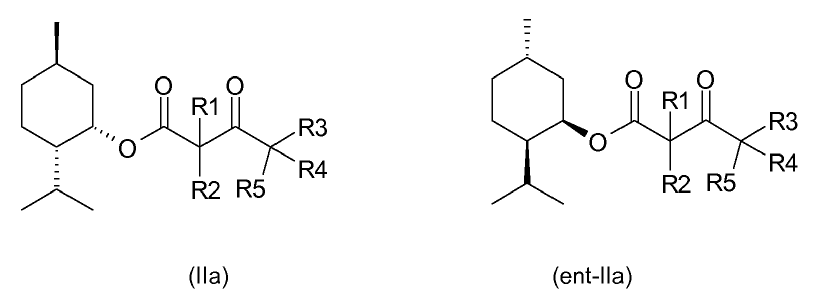 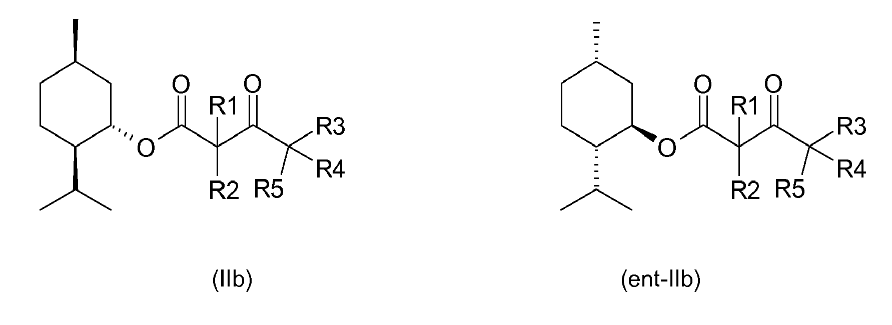 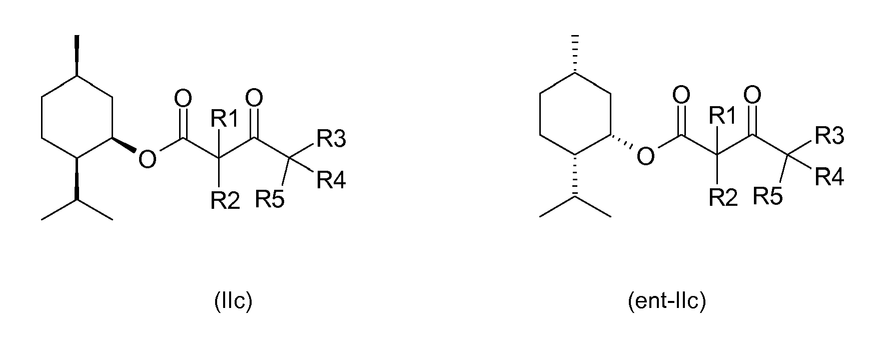
wobei in jeder der Formeln (IIa), (IIb), (IIc), (ent-IIa), (ent-IIb) und (ent-IIc) R1, R2, R3, R4 und R5 jeweils unabhängig voneinander eine der in einem der vorangehenden Ansprüche für die Formeln (I) und (ent-I) angegebenen Bedeutungen besitzen,
wobei die jeweiligen Bedeutungen von R1, R2, R3, R4 und R5 für die in der Mischung vorliegenden Verbindungen der Formeln (I), (ent-I), (IIa), (IIb), (IIc), (ent-IIa), (ent-IIb) und (ent-IIc) jeweils voneinander unabhängig sind,
- als Bestandteil (c) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
- als Bestandteil (d) einen oder mehrere Aromastoffe ohne physiologische Kühlwirkung
- als Bestandteil (e) einen oder mehrere trigeminal oder mundwässernd wirksame Stoffe ohne physiologische Kühlwirkung,
wobei, sofern als erste Verbindung der Formel (I) L-Menthyl-3-oxobutyrat und als oder in Bestandteil (c) eine eine physiologische Kühlwirkung erzeugende Menge an L-Menthyl-3-hydroxybutyrat eingesetzt wird, die Mischung kein Reduktionsmittel für L-Menthyl-3-oxobutyrat umfasst.

7. Mischung nach Anspruch 6, wobei in einer, zwei, drei, mehr als drei oder sämtlichen in der Mischung enthaltenen Verbindungen (I), (ent-I), (IIa), (IIb), (IIc), (ent-IIa), (ent-IIb) und (ent-IIc) R1, R2, R3, R4 und R5 die in einem der Ansprüche 2, 3, 4 oder 5 für die Formeln (I) und (ent-I) angegebenen Bedeutungen besitzen.

8. Mischung nach einem der Ansprüche 6 bis 7, als oder in Bestandteil (b) umfassend eine Verbindung oder ein Gemisch von Verbindungen der Formeln (IIa), (IIb) und/oder (IIc).

9. Mischung nach einem der Ansprüche 6 bis 8, wobei das Gewichtsverhältnis von (a) der Gesamtheit von Verbindungen der Formel (I) und (ent-I) zu (b) der Gesamtheit von Verbindungen der Formeln (IIa), (IIb), (IIc), (ent-IIa), (ent-IIb) und (ent-IIc) im Bereich von 200 : 1 bis 4 : 1, bevorzugt im Bereich von 100 : 1 bis 10 : 1, insbesondere bevorzugt im Bereich von 100 : 1 bis 20 : 1 liegt.

10. Mischung nach einem der Ansprüche 6 bis 9, umfassend eine oder mehrere Verbindungen der Formel (I), wobei der Anteil von Verbindungen der Formel (I) an dem Gesamtgewicht von Verbindungen der Formeln (I), (ent-I), (IIa), (IIb), (IIc), (ent-IIa), (ent-IIb) und (ent-IIc) zumindest 90 Gew.-% beträgt, vorzugsweise zumindest 95 Gew.-%.

11. Mischung nach einem der Ansprüche 6 bis 10, umfassend in oder als Bestandteil (c)
- einen oder mehrere Stoffe mit physiologischer Kühlwirkung, wobei diese keinen geschmacklichen Effekt und keine Aromawirkung verursachen oder
- eine, zwei oder mehr Verbindungen ausgewählt aus der Gruppe bestehend aus: Menthol und Mentholderivate, Menthylether, Menthylester, Menthylcarbonate, Halbester von Mentholen mit einer Dicarbonsäure und deren Derivate, Menthancarbonsäureamide, Menthon und Menthonderivate, 2,3-Dimethyl-2-(2-propyl)-butansäurederivate, Isopulegol und seine Ester, Cubebol, synthetische oder natürliche Mischungen enthaltend Cubebol, Pyrrolidonderivate von Cycloalkyldionderivaten und Tetrahydropyrimidin-2-one.

12. Mischung nach einem der Ansprüche 6 bis 11, umfassend 0,05 bis 90 Gew.-% des Bestandteils (a) sowie 0,01 bis 90 Gew.-% des Bestandteils (c), bezogen auf das Gesamtgewicht der Bestandteile (a), (b), (c), (d) und (e).

13. Mischung nach einem der Ansprüche 6 bis 12, umfassend als oder in Komponente (d) einen oder mehrere Aromastoffe ohne physiologische Kühlwirkung, der bzw. die einen Geschmackseindruck, einen geschmacksmodulierenden Effekt, einen trigeminalen Effekt und/oder einen mundwässernden Reiz verursacht bzw. verursachen.

14. Mischung nach Anspruch 13, umfassend als oder in Komponente (d) einen oder mehrere Aromastoffe ohne physiologische Kühlwirkung, der bzw. die verursachen:
- einen oder mehrere Geschmackseindrücke aus der Gruppe bestehend aus süß, Umami, bitter, salzig und sauer und/oder
- einen oder mehrere geschmacksmodulierende Effekte aus der Gruppe bestehend aus: bitter-maskierend, umami-verstärkend, süß-verstärkend, salz-verstärkend und sauer-maskierend
und/oder
- einen oder mehrere trigeminale Reize aus der Gruppe bestehend aus:
Schärfe, Wärme, Kribbeln und Stechen
sowie gegebenenfalls
- einen mundwässernden Reiz.

15. Der Ernährung, der Mundhygiene oder dem Genuss dienende oder pharmazeutische oder kosmetische Zubereitung, umfassend eine zum Erreichen einer physiologischen Kühlwirkung auf der Haut und/oder Schleimhaut ausreichende Menge einer Verbindung der Formel (I) oder (ent-I) oder einer Mischung bestehend aus zwei, drei oder mehr Verbindungen der Formel (I) oder (ent-I) wie in einem der Ansprüche 1 bis 5 definiert.

16. Zubereitung nach Anspruch 15, umfassend oder bestehend aus einer Mischung nach einem der Ansprüche 6 bis 14, wobei, sofern in der Mischung als erste Verbindung der Formel (I) L-Menthyl-3-oxobutyrat und als oder in Bestandteil (c) eine eine physiologische Kühlwirkung erzeugende Menge an L-Menthyl-3-hydroxybutyrat eingesetzt wird, die Zubereitung kein Reduktionsmittel für L-Menthyl-3-oxobutyrat umfasst.

17. Zubereitung nach einem der Ansprüche 14 oder 15, umfassend
- insgesamt 0,0001 Gew.-% bis 20 Gew.-%, bevorzugt 0,0001 bis 10 Gew.-%, besonders bevorzugt 0,001 Gew.-% bis 0,5 Gew.-% an Verbindungen der Formel (I), bezogen auf das Gesamtgewicht der Zubereitung,
- insgesamt 0,0000001 bis 99,99 Gew.-%, vorzugsweise 10 bis 80 Gew.-% an Verbindungen der Formel (ent-I), Bestandteilen (b), (c), (d) und/oder (e) wie in einem der Ansprüche 6 bis 14 definiert sowie weiteren Grund-, Hilfs- und Zusatzstoffen mit Ausnahme von Wasser, bezogen auf das Gesamtgewicht der Zubereitung,
- 0 bis 99,99 Gew.-% Wasser, bezogen auf das Gesamtgewicht der Zubereitung, vorzugsweise 5 bis 80 Gew.-%.

18. Der Ernährung oder dem Genuss dienende Zubereitung nach einem der Ansprüche 15 bis 17, ausgewählt aus der Gruppe bestehend aus: Backwaren, Süßwaren, alkoholische oder nicht-alkoholische Getränke, Instantgetränke, Fleischprodukte, Eier oder Eiprodukte , Getreideprodukte, Milchprodukte, Fruchtzubereitungen, Gemüsezubereitungen, Knabberartikel, Produkte auf Fett- und Ölbasis oder Emulsionen derselben, sonstige Fertiggerichte und Suppen, Gewürze, Würzmischungen, Aufstreuwürzungen, Halbfertigwaren, Nahrungsergänzungsmittel.

19. Der Mundhygiene dienende Zubereitung nach einem der Ansprüche 15 bis 17, auf Basis eines Zahnpflegemittels und ausgewählt aus der Gruppe bestehend aus: Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide und Zahnpflegekaugummi.

20. Pharmazeutische Zubereitung nach einem der Ansprüche 15 bis 17, wobei die Zubereitung eine orale pharmazeutische Zubereitung ist, vorzugsweise in Form von Kapseln, Tabletten, Dragees, Granulaten, Pellets, Feststoffmischungen, Dispersionen in flüssigen Phasen, als Emulsionen, als Pulver, als Lösungen, als Pasten oder als andere schluck- oder kaubare Zubereitung.

21. Kosmetische Zubereitung nach einem der Ansprüche 15 bis 17, ausgewählt aus der Gruppe bestehend aus: Seife, Syndet, flüssiges Wasch-, Dusch-, oder Badepräparat, Emulsion, Salbe, Paste, Gel, Öl, Toner, Balsam, Serum, Puder, Eau de Toilette, Toilette, Eau de Cologne, Perfum, Wachs, Stift, Roll-On, (Pump-)Spray, Aerosol (schäumend, nicht schäumend oder nach-schäumend), Fusspflegemittel, Bartreinigungs- oder -pflegemittel, Insekten abwehrendes Mittel, Sonnenschutzmittel, Aftersun-Präparat, Rasiermittel, After-Shave, Haarentfernungsmittel, Haarpflegemittel, Conditioner, Haarkur, Haarwasser, Haarspülung, Frisiercreme, Pomade, Dauerwell- und Fixierungsmittel, Haarglättungsmittel, Haarfestiger, Styling-Hilfe, Blondiermittel, Haaraufheller, Haarconditioner, Haarmousse, Haartönung, Nagelpflegemittel, Deodorant, Antitranspirant, Mundwasser, Munddusche, Make-Up, Make-Up-Entferner, Augenpflege, Lippenkosmetika, Lippenpflegemittel, dekorative Kosmetik, Badeartikel und Maske.

22. Therapeutisches oder nicht-therapeutisches Verfahren zum Erreichen einer physiologischen Kühlwirkung auf der Haut und/oder Schleimhaut, umfassend den folgenden Schritt:
- Applizieren einer zum Erreichen einer physiologischen Kühlwirkung ausreichenden Menge
(i) einer Verbindung der Formel (I) oder (ent-I) oder einer Mischung bestehend aus zwei, drei oder mehr Verbindungen der Formel (I) oder (ent-I) wie in einem der Ansprüche 1 bis 5 definiert, oder
(ii) einer Mischung nach einem der Ansprüche 6 bis 14, oder
(iii) einer Zubereitung nach einem der Ansprüche 15 bis 21
auf die Haut und/oder Schleimhaut.
